# EUROPEAN PATENT APPLICATION

(11) **EP 3 977 894 A1**
(43) Date of publication of application: **06.04.2022**
(21) Application number: 19931293.5
(22) Date of filing: 28.05.2019
(51) Int. Cl.: A46B 15/00, A46B 5/00

(54) **TOOTHBRUSH HAVING PHYSIOLOGICAL SENSING FUNCTION**

(71) Applicant: Acumen Houseware Industry (Nanning) Co., Ltd, Nanning, Guangxi 530221 (CN)
(72) Inventor: HUANG, Shou Jen, Taipei City, Taiwan (CN)
(74) Representative: Viering, Jentschura & Partner mbB Patent- und Rechtsanwälte
(86) International application number: PCT/CN2019/088754
(87) International publication number: WO 2020/237494

(57) **Abstract**

A toothbrush (10) has a holding segment (11), a sensing module (12), and a brush head segment (13). The holding segment (11) has an inner space, a top end (1101), a bottom end (1102), and a sensing area (1103). The sensing module (12) is mounted in the inner space of the holding segment (11) and corresponds in position to the sensing area (1103). The sensing module (12) runs a physiological detecting function at the sensing area (1103) and generates a physiological data. The brush head segment (13) is mounted on the top end (1101) of the holding segment (11). When a user brushes teeth with the toothbrush (10), a finger pad or a palm of the user touches the sensing area (1103), and the sensing module (12) runs the physiological detecting function and then generates physiological data.

## Description

### 1. Field of the Invention

The present invention relates to a toothbrush, especially to a toothbrush with a physiological detecting function.

### 2. Description of the Prior Arts

Recent years, wearable devices are getting more and more popular in the field of consumer electronics. A wearable device not only works in connection with a cellphone but also detects the physiological state of the user because it is light and close-fitting, thereby allowing the users to record their physical health conditions at any time.

However, when brushing teeth, a user might not wear any wearable device because brushing teeth is usually done before or after taking a shower, before going to bed, or right after getting up, and the user might want to feel comfortable and take off the wearable device. Thus, the user cannot obtain information about his/her physical state in real time in this situation, which is inconvenient.

To overcome the shortcomings, the present invention provides a toothbrush to mitigate or obviate the aforementioned problems.

The main objective of the present invention is to provide a toothbrush that is capable of detecting the physiological state of the user.

The toothbrush has a holding segment, a sensing module, and a brush head segment. The holding segment has an inner space, a top end, a bottom end, and a sensing area. The bottom end is opposite to the top end. The bottom end and the top end are arranged along a lengthwise direction of the holding segment. The sensing module is mounted in the inner space of the holding segment and corresponds in position to the sensing area. The sensing module runs a physiological detecting function at the sensing area and generates a physiological data. The brush head segment is mounted on the top end of the holding segment.

When a user brushes his/her teeth with the toothbrush of the present invention, a finger pad or a palm of the user touches the sensing area, and the sensing module runs the physiological detecting function and then generates physiological data. Then, the physiological data can be sent to an external processing module to run health management analysis or be shown to the user. Therefore, the user can understand his/her own health information more comprehensively.

Other objectives, advantages and novel features of the invention will become more apparent from the following detailed description when taken in conjunction with the accompanying drawings.

### IN THE DRAWINGS

Fig. 1 is a perspective view of a toothbrush in accordance with the present invention;
Fig. 2 is an exploded view of the toothbrush in Fig. 1; and
Fig. 3 is another exploded view of the toothbrush in Fig. 1.

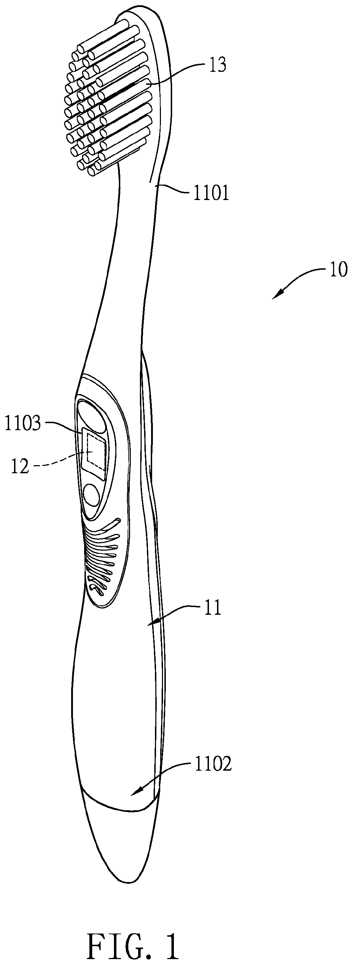

With reference to Fig. 1, a toothbrush 10 in accordance with the present invention comprises a holding segment 11, a sensing module 12, and a brush head segment 13. The holding segment 11 has an inner space, a top end 1101, a bottom end 1102, and a sensing area 1103. The top end 1101 and the bottom end 1102 are arranged along a lengthwise direction of the holding segment 11 and are opposite to each other. The sensing area 1103 is formed on a surface of the holding segment. The sensing module 12 is mounted in the inner space of the holding segment 11 and corresponds in position to the sensing area 1103. The sensing module 12 runs a physiological detecting function and generates a physiological data. The brush head segment 13 is mounted on the top end 1101 of the holding segment 11.

The physiological detecting function run by the sensing module 12 comprises heart rhythm sensing, blood oxygen sensing, blood pressure sensing, or body temperature sensing. The sensing module 12 can be implemented as an optical pulse detection module, an infrared blood oxygen sensing module, a blood pressure sensing module or a temperature sensing module, etc.

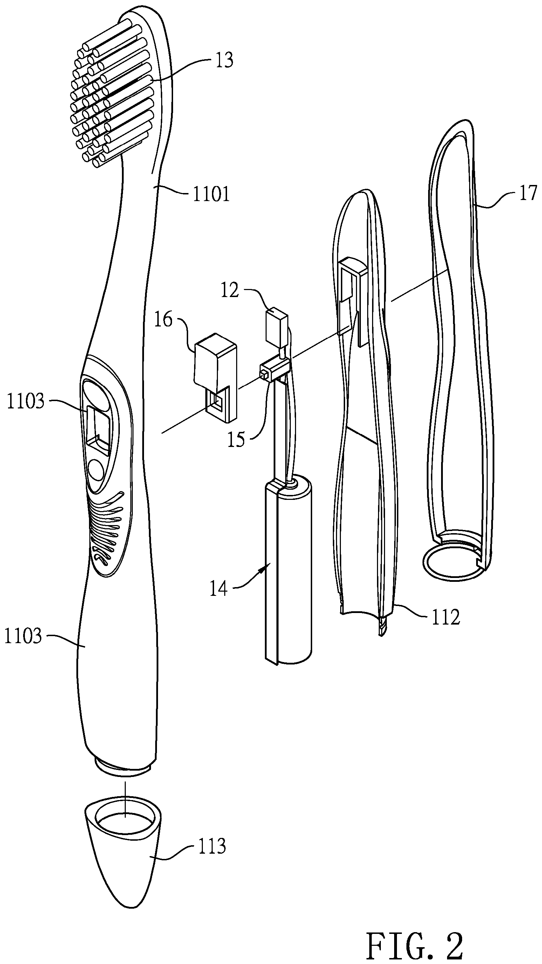

With reference to Fig. 2, in a first embodiment, the toothbrush 10 has a power module 14. The power module 14 is also mounted in the inner space of the holding segment 11, is electrically connected to the sensing module 12, and supplies power to the sensing module 12. The power module 14 can be implemented as a replaceable battery module or a rechargeable battery module.

With reference to Fig. 2, preferably, the toothbrush 10 has a switch 15. The power module 14 is electrically connected to the sensing module 12 through the switch 15. The switch 15 has an on state and an off state. When the switch 15 is in the on state, the power module 14 supplies power to the sensing module 12 through the switch 15. When the switch 15 is in the off state, the power module 14 cannot supply power to the sensing module 12 such that the sensing module 12 cannot run the physiological detecting function. Preferably, the switch 15 is mounted on the holding segment 11 to facilitate user operation so that the user can easily control the power module 14 to supply power to the sensing module 12 for running the physiological detecting function.

In a second embodiment, the toothbrush 10 has a sensing cover 16, and the holding segment 11 has a sensing area opening formed on the sensing area 1103. The sensing area opening communicates with the inner space. The sensing cover 16 is mounted in the sensing area opening and the sensing module 12 corresponds in position to the sensing cover 16. The sensing cover 16 protects the sensing module 12. The material of the sensing cover 16 can be determined according to the type of the sensing module 12, such as a transparent cover for optical physiological sensing, or a silicone elastic cover for pressure sensing, etc.

Preferably, the sensing module 12 has a sensing unit and a wireless communication unit. The sensing unit runs the physiological detecting function and generates the physiological data. The wireless communication unit is electrically connected to the sensing unit to receive the physiological data generated by the sensing unit. The wireless communication unit is wirelessly connected to an external processing module and sends the physiological data to the external processing module.

The external processing module can be implemented as a cloud server, a wireless receiving and display module, or a smart mobile device. In the case of the smart mobile device, the smart mobile device is wirelessly connected to the wireless communication unit of the toothbrush 10. The smart mobile device receives the physiological data generated by the sensing unit via the wireless communication unit, and then performs data analysis, such as curve analysis, abnormal surge value analysis, etc., and provides results of analysis to the user through the display screen to remind the user to pay attention to his/her health.

With reference to Fig. 1, in a third embodiment, the holding segment 11 has a front side and a back side arranged along a thickness direction and being opposite to each other. The sensing area 1103 is preferably mounted on the front side of the holding segment 11, so as to conform to the holding posture of general users, which is clamping the holding segment 11 by the thumb and index finger front and back. Therefore, when brushing teeth, the thumb or the index finger of the user will touch the sensing area 1103 so that the sensing module 12 can run the physiological detecting function. The sensing area 1103 can also be mounted on the back side, or there are multiple said sensing areas 1103 respectively configured with multiple said sensing modules 12 so as to increase the number of sensing samples and improve the accuracy of sensing. The bristles of the brush head segment 13 extend toward the front side.

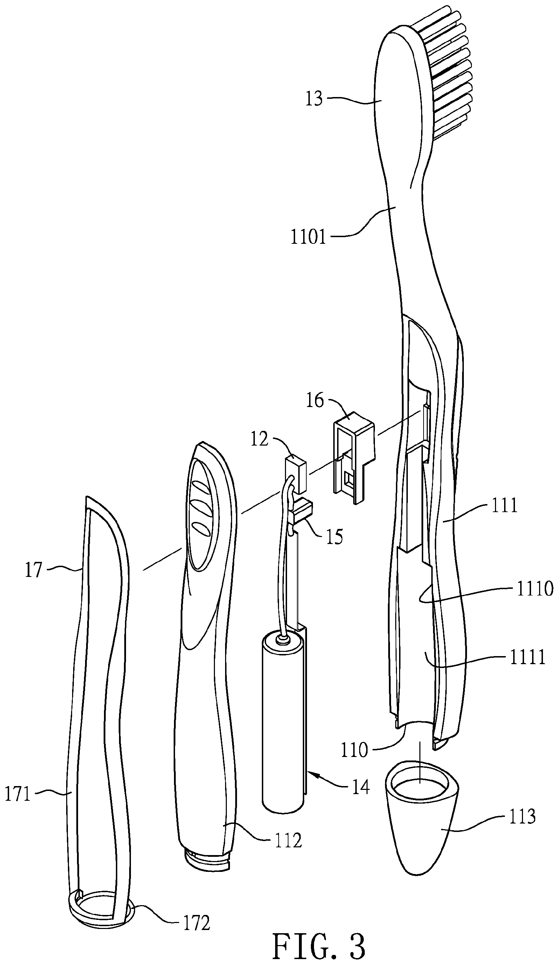

With reference to Figs. 2 and 3, in a fourth embodiment, a main body 111 and a back cover 112 are assembled to form the holding segment 11. The main body 111 faces toward the front side, and the back cover 112 faces toward the back side. The main body 111 has an accommodating groove 1111. The sensing module 12 and the power module 14 are mounted in the accommodating groove 1111. The accommodating groove 1111 has a first opening 1110, and the first opening 1110 faces toward the back side. A peripheral contour of the back cover 112 conforms to that of the first opening 1110. The back cover 112 is mounted on a periphery of the first opening 1110 to cover and seal the first opening 1110 and is assembled with the main body 111 to form the holding segment 11. After the main body 111 and the back cover 112 are assembled, the accommodating groove 1111 of the main body 111 forms the inner space of the holding segment 11.

In a fifth embodiment, the holding segment 11 has a bottom end opening 110 and a bottom cover 113, and the power module 14 is implemented as a replaceable battery module. The bottom end opening 110 communicates with the inner space, and the bottom cover 113 is sleeved on the bottom end 1102 to cover the bottom end opening 110. The bottom cover 113 can be detached for the user to replace the battery in the inner space. The replaceable battery can be carbon-zinc batteries, alkaline batteries, lithium batteries, etc.

Preferably, the holding segment 11 has a sealing unit 17 mounted between the main body 111, the back cover 112, and the bottom cover 113 to seal the gap between the main body 111, the back cover 112, and the bottom cover 113 and to ensure water resistance during use. The sealing unit 17 is preferably a sealing gasket made of rubber. The sealing unit 17 has a first opening sealing segment 171. The periphery of the first opening 1110 and the periphery of the back cover 112 are both concaved to form an engaging groove. The engaging groove extends along the periphery of the first opening 1110 and the periphery of the back cover 112. When assembling the sealing unit 17, the first opening sealing segment 171 of the sealing unit 17 is mounted into the engaging groove and is tightly fit in the engaging groove. The sealing unit 17 covers the junction between the periphery of the first opening 1110 and the periphery of the back cover 112 in order to prevent liquid from leaking into the inner space of the holding segment 11 and damaging the power module 14 or the sensing module 12. Further, the sealing unit 17 has a bottom end opening sealing segment 172 sleeved on an outer wall of the bottom end 1102 of the holding segment 11. When the bottom cover 113 covers the bottom end 1102, the bottom end opening sealing segment 172 is tightly mounted between an outer wall of the holding segment 11 and an inner wall of the bottom cover 113 to prevent liquid from leaking into the inner space of the holding segment 11.

## Claims

1. A toothbrush (10) **characterized in that** the toothbrush (10) comprises:
a holding segment (11) having
an inner space;
a top end (1101);
a bottom end (1102) being opposite to the top end (1101); the bottom end (1102) and the top end (1101) arranged along a lengthwise direction of the holding segment (11); and
a sensing area (1103);
a sensing module (12) mounted in the inner space of the holding segment (11) and corresponding in position to the sensing area (1103); the sensing module (12) running a physiological detecting function at the sensing area (1103) and generating a physiological data; and
a brush head segment (13) mounted on the top end (1101) of the holding segment (11).

2. The toothbrush (10) as claimed in claim 1, wherein the physiological detecting function run by the sensing module (12) comprises heart rhythm sensing, blood oxygen sensing, blood pressure sensing, or body temperature sensing.

3. The toothbrush (10) as claimed in claim 1 or 2, wherein
the toothbrush (10) has
a power module (14) mounted in the inner space of the holding segment (11), electrically connected to the sensing module (12), and supplying power.

4. The toothbrush (10) as claimed in claim 3, wherein
the toothbrush (10) has
a switch (15) having an on state and an off state; and
the power module (14) is electrically connected to the sensing module (12) through the switch (15).

5. The toothbrush (10) as claimed in claim 1 or 2, wherein
the sensing area (1103) of the holding segment (11) has
a sensing area (1103) opening communicating with the inner space;
the toothbrush (10) has
a sensing cover (16) mounted in the sensing area (1103) opening; and
the sensing module (12) corresponds in position to the sensing cover (16) and runs the physiological detecting function via the sensing cover (16).

6. The toothbrush (10) as claimed in claim 4, wherein
the sensing module (12) has
a sensing unit running the physiological detecting function and generating the physiological data; and
a wireless communication unit electrically connected to the sensing unit to receive the physiological data generated by the sensing unit; the wireless communication unit wirelessly connected to an external processing module and sending the physiological data to the external processing module.

7. The toothbrush (10) as claimed in claim 1 or 2, wherein
the holding segment (11) has
a front side;
a back side being opposite to the back side; the back side and the front side arranged along a thickness direction of the holding segment (11); and
the sensing area (1103) is mounted on the front side.

8. The toothbrush (10) as claimed in claim 3, wherein
the holding segment (11) has
a main body (111) having
an accommodating groove (1111); the sensing module (12) and the power module (14) mounted in the accommodating groove (1111); the accommodating groove (1111) having
a first opening (1110); and
a back cover (112) assembled with the main body (111) and covering the first opening (1110); wherein after the back cover (112) and the main body (111) are assembled, the accommodating groove (1111) of the main body (111) forms the inner space of the holding segment (11).

9. The toothbrush (10) as claimed in claim 8, wherein
the bottom end (1102) of the holding segment (11) has
a bottom end (1102) opening communicating with the inner space; and
the holding segment (11) has
a bottom cover (113) covering the bottom end (1102) opening.

10. The toothbrush (10) as claimed in claim 9, wherein
the holding segment (11) has
a sealing unit (17) mounted between a periphery of the first opening (1110) of the main body (111) and a periphery of the back cover (112), and mounted between the bottom end (1102) of the holding segment (11) and the bottom cover (113).
